# EUROPEAN PATENT APPLICATION

(11) **EP 3 243 856 A1**
(43) Date of publication of application: **15.11.2017**
(21) Application number: 15872204.1
(22) Date of filing: 15.12.2015
(51) Int. Cl.: C08G 59/46, C08L 63/00

(54) **EPOXY RESIN COMPOSITION**

(30) Priority: 22.12.2014 JP 2014259256
(71) Applicant: Nippon Soda Co., Ltd., Tokyo 100-8165 (JP)
(72) Inventor: ONO, Kazuo, Ichihara-shi Chiba 290-0045 (JP)
(74) Representative: Wills, Andrew Jonathan
(86) International application number: PCT/JP2015/006241
(87) International publication number: WO 2016/103630

(57) **Abstract**

It is to provide an epoxy resin comprising a phenol compound having a curing acceleration effect even when a low reactive curing agent is used, and allowing the amount of the curing agent used to be reduced. Here is used an epoxy resin composition comprising a glycidylamine-type epoxy resin, an aromatic polyamine and 1,1,2,2-tetrakis(4-hydroxyphenyl)ethane. The aromatic polyamine and 1,1,2,2-tetrakis(4-hydroxyphenyl)ethane optionally form a clathrate compound.

## Description

### Technical Field

The present invention relates to an epoxy resin composition excellent in the thermal properties during curing, and a novel clathrate compound. The present application claims the priority of Japanese Patent Application No. 2014-259256 filed December 22, 2014, the contents of which are cited herein to form part of the present application.

### Background Art

A resin composition comprising a glycidylamine-type epoxy resin and an aromatic polyamine is excellent in heat resistance and modulus of elasticity of the cured product thereof, and accordingly has been frequently used as a matrix resin of a fiber-reinforced composite material. In general, the aromatic polyamine is known to be slow in the progress of a cross-linking reaction, and accordingly frequently used by mixing therein a curing accelerator such as a tertiary amine, a Lewis acid complex, an onium salt, imidazole or a phenol compound.

As an example of using a phenol compound as a curing accelerator, there has been known an epoxy resin composition for a fiber-reinforced composite material consisting of 100 parts by mass of tetraglycidyl diaminodiphenylmethane (hereinafter, also referred to as TGDDM), 43.4 parts by mass of an aromatic polyamine curing agent (parts by mass ratio: 70:15:15) consisting of diethyl toluene diamine, 4,4'-diaminodiphenyl sulfone (hereinafter, also referred to as 4,4'-DDS) and 3,3'-diaminodiphenyl sulfone (hereinafter, also referred to as 3,3'-DDS), and 1.0 part by mass of 4-tert-butylcatechol (TBC) as a curing accelerator (see Patent Literature 1).

### Prior Art Document

### Patent Document

Patent Document 1: WO2003/040206

### Summary of the Invention

### Object to be Solved by the Invention

Only TBC is known as a practical phenol compound as a curing accelerator in the case where a low reactive curing agent such as 4,4'-DDS is used, and there has been a problem that even when TBC is used, a curing agent is required to be used in a stoichiometric amount, and the handling is difficult.

An object of the present invention is to provide an epoxy resin composition comprising a phenol compound having a curing acceleration effect even when a low reactive curing agent is used, and allowing the amount of the curing agent used to be reduced.

### Means to Solve the Object

The present inventors made a diligent study in order to achieve the above-described object, consequently have discovered that the above-described object may be achieved by adding 1,1,2,2-tetrakis(4-hydroxyphenyl)ethane (hereinafter, sometimes abbreviated as TEP) to a composition comprising a glycidylamine-type epoxy resin and an aromatic polyamine, and thus have completed the present invention.

Specifically, the present invention relates to the following:
(1) an epoxy resin composition comprising a glycidylamine-type epoxy resin, an aromatic polyamine and 1,1,2,2-tetrakis(4-hydroxyphenyl)ethane;
(2) the epoxy resin composition according to (1), wherein the aromatic polyamine and 1,1,2,2-tetrakis(4-hydroxyphenyl)ethane form a clathrate compound;
(3) the epoxy resin composition according to (1) or (2), wherein the aromatic polyamine is 4,4'-diaminodiphenyl sulfone and/or 3,3'-diaminodiphenyl sulfone;
(4) the epoxy resin composition according to (1) or (2), wherein the glycidylamine-type epoxy resin is a tri-or higher functional glycidylamine-type epoxy resin;
(5) the epoxy resin composition according to (4), wherein the tri- or higher functional glycidylamine-type epoxy resin is N,N,N',N'-tetraglycidyl-4,4'-diaminodiphenylmethane; and
(6) a cured product obtained by curing the epoxy resin composition according to any one of (1) to (5).

Moreover, the present invention relates to
(7) a clathrate compound comprising (A) 4,4'-diaminodiphenyl sulfone and/or 3,3'-diaminodiphenyl sulfone and (B) 1,1,2,2-tetrakis(4-hydroxyphenyl)ethane.

### Effect of the Invention

The use of the epoxy resin composition of the present invention enables the reduction of the heat quantity and the decrease of the curing temperature during curing, accordingly enables the curing with a smaller energy as compared with an epoxy resin composition not including TEP as added therein, and moreover, enables to efficiently obtain a cured product having physical properties equivalent to the conventional physical properties with a curing agent of a quantity equal to or less than the stoichiometric quantity. The epoxy resin composition of the present invention comprising the aromatic polyamine included with TEP is excellent with respect to the one-pack stability, as compared with an epoxy resin composition comprising an aromatic polyamine not included with TEP.

### Brief Description of Drawings

[Figure 1] Figure 1 is a chart showing the results obtained by performing a thermogravimetric measurement·differential scanning calorimetry (sometimes referred to as TG-DSC) of a clathrate compound (A-1).
[Figure 2] Figure 2 is a chart showing the results obtained by measuring the TG-DSC of a clathrate compound (A-2).
[Figure 3] Figure 3 is a chart showing the results obtained by performing a differential scanning calorimetry (sometimes referred to as DSC) of epoxy resin compositions (B-1), (B-5) and (CB-1).
[Figure 4] Figure 4 is a chart showing the results obtained by measuring the DSC of epoxy resin compositions (B-4), (B-10) and (CB-4).

### Mode of Carrying Out the Invention

### (Glycidylamine-Type Epoxy Resin)

The glycidylamine-type epoxy resin is not particularly limited as long as the glycidylamine-type epoxy resin is a compound intramolecularly having a glycidylamino group or a glycidylimino group; however, specifically, as the glycidylamine-type epoxy resin, the compounds listed below or the like may be exemplified. Because the mechanical properties, the heat resistance, the environment resistance and the like of the obtained cured product are excellent, a tri- or higher functional glycidylamine-type epoxy resin is preferable, and moreover, tetraglycidyl diaminodiphenylmethane is preferable.

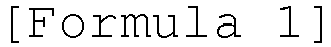

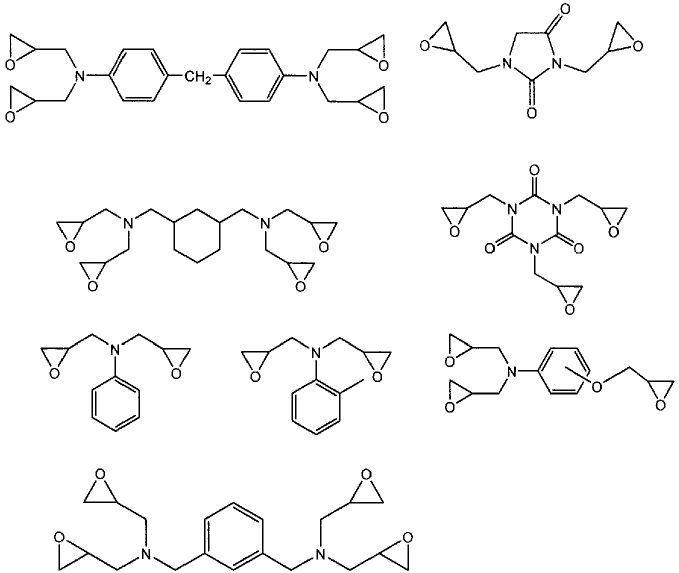

As the commercially available products of tetraglycidyl diaminodiphenylmethane, Sumiepoxy (registered trademark) ELM434 (manufactured by Sumitomo Chemical Co., Ltd.); "Araldite (registered trademark)" MY720, "Araldite (registered trademark)"MY721, "Araldite (registered trademark)" MY9512, "Araldite (registered trademark)" MY9663 (these are manufactured by Huntsman Advanced Materials Co., Ltd.); jER (registered trademark) 604 (manufactured by Mitsubishi Chemical Corp.); "Epotohto (registered trademark) "YH-434 (manufactured by Nippon Steel Chemical Co., Ltd.); or the like may be exemplified.

### (Other Epoxy Resins)

In the epoxy resin composition of the present invention, it is possible to mix an epoxy resin (hereinafter, also referred to as a "polyfunctional epoxy resin") having two or more epoxy groups in one molecule thereof, other than the above-described glycidylamine-type epoxy resin. Herein, the epoxy resin means a prepolymer before curing, and includes monomers and oligomers. Specifically,
novolac-type epoxy resins such as phenol novolac-type epoxy resins and orthocresol novolac-type epoxy resins obtained by epoxidizing novolac resins obtained by condensing or cocondensing in the presence of an acidic catalyst at least one phenolic compound selected from the group consisting of phenol compounds such as phenol, cresol, xylenol, resorcin, catechol, bisphenol A and bisphenol F and naphthol compounds such as α-naphthol, β-naphthol and dihydroxynaphthalene and aliphatic aldehyde compounds such as formaldehyde, acetaldehyde and propionaldehyde;
triphenylmethane-type epoxy resins obtained by epoxidizing triphenylmethane-type phenolic resins obtained by condensing or cocondensing in the presence of an acidic catalyst the above described phenolic compounds and aromatic aldehyde compounds such as benzaldehyde and salicylaldehyde;
copolymerization-type epoxy resins obtained by epoxidizing the novolac resins obtained by cocondensing in the presence of an acidic catalyst the above-described phenol compounds, naphthol compounds and aldehyde compounds;
diphenylmethane-type epoxy resins being diglycidyl ethers of bisphenol A, bisphenol F and the like;
biphenyl-type epoxy resins being diglycidyl ethers of alkyl-substituted or non-alkyl-substituted biphenyls;
stilbene-type epoxy resins being diglycidyl ethers of stilbene-based phenol compounds;
sulfur atom-containing epoxy resins being diglycidyl ethers of bisphenol S and the like;
epoxy resins being glycidyl ethers of alcohols such as butane diol, polyethylene glycol and polypropylene glycol;
glycidyl ester-type epoxy resins of polyvalent carboxylic acid compounds such as phthalic acid, isophthalic acid and tetrahydrophthalic acid;
glycidylamine-type epoxy resins obtained by substituting, with a glycidyl group, the active hydrogen bonded to the nitrogen atom in aniline, diaminodiphenylmethane, isocyanuric acid and the like;
dicyclopentadiene-type epoxy resins obtained by epoxidizing the cocondensed resins of dicyclopentadiene and phenol compounds;
alicyclic type epoxy resins obtained by epoxidizing the intramolecular olefin bonds such as vinylcyclohexene diepoxide, 3,4-epoxy cyclohexyl methyl-3,4-epoxy cyclohexane carboxylate and 2-(3,4-epoxy)cyclohexyl-5,5-spiro(3,4-epoxy)cyclohexane-m-dioxane;
glycidyl ethers of paraxylylene-modified phenol resins;
glycidyl ethers of metaxylylene-modified phenol resins;
glycidyl ethers of terpene-modified phenol resins;
glycidyl ethers of dicyclopentadiene-modified phenol resins;
glycidyl ethers of cyclopentadiene-modified phenol resins;
glycidyl ethers of polycyclic aromatic ring-modified phenol resins;
naphthalene-type epoxy resins being glycidyl ethers of naphthalene ring-containing phenol resins;
halogenated phenol novolac-type epoxy resins;
hydroquinone-type epoxy resins;
trimethylolpropane-type epoxy resins;
linear aliphatic epoxy resins obtained by oxidizing olefin bonds with peracids such as peracetic acid;
diphenylmethane-type epoxy resins;
aralkyl-type epoxy resins being epoxidized products of aralkyl-type phenol resins such as phenol aralkyl resins and naphthol aralkyl resins; or the like may be exemplified. These may be used alone or by combination of two or more thereof.

### (Aromatic Polyamines)

The aromatic polyamines used in the epoxy resin composition of the present invention are the compounds having a structure in which an amino group having an active hydrogen is directly bonded to an aromatic ring and having in one molecule two or more active hydrogen atoms of such amino groups.

Specifically, 4,4'-methylenedianiline, 4,4'-methylenebis(2-methylaniline), 4,4'-methylenebis(2-ethylaniline), 4,4'-methylenebis(2-isopropylaniline), 4,4'-methylenebis(2-chloroaniline), 4,4'-methylenebis(2,6-dimethylaniline), 4,4'-methylenebis(2,6-diethylaniline), 4,4'-methylenebis(2-isopropyl-6-methylaniline), 4,4'-methylenebis(2-ethyl-6-methylaniline), 4,4'-methylenebis(2-bromo-6-ethylaniline), 4,4'-methylenebis(N-methylaniline), 4,4'-methylenebis (N-ethylaniline), 4,4'-methylenebis (N-sec-butyl aniline), 4,4'-diaminodiphenyl sulfone, 3,3'-diaminodiphenyl sulfone, 4,4'-cyclohexylidenedianiline, 4,4'-(9-fluorenylidene)dianiline, 4,4'-(9-fluorenylidene)bis(N-methylaniline), 4,4'-diaminobenzanilide, 4,4'-oxydianiline, 2,4-bis(4-aminophenylmethyl)aniline, 4-methyl-m-phenylenediamine, 2-methyl-m-phenylenediamine, N,N'-di-sec-butyl-p-phenylenediamine, 2-chloro-p-phenylenediamine, 2,4,6-trimethyl-m-phenylenediamine, diethyl toluene diamine (a mixture mainly consisting of 2,4-diethyl-6-methyl-m-phenylenediamine and 4,6-diethyl-2-methyl-m-phenylenediamine), bis(methylthio)toluene diamine (a mixture mainly consisting of 6-methyl-2,4-bis(methylthio)-m-phenylenediamine and 2-methyl-4,6-bis (methylthio)-m-phenylenediamine), 4,6-dimethyl-m-phenylenediamine, trimethylene bis(4-aminobenzoate) or the like may be exemplified. Among these, because of being high in heat resistance and low in melting point, it is preferable to use 4,4'-diaminodiphenylmethane and/or 3,3'-diaminodiphenylmethane. These compounds may be used alone or used as mixtures of two or more thereof.

### (Clathrate Compound)

The epoxy resin composition of the present invention comprises TEP and an aromatic polyamine; TEP and the aromatic polyamide may be each used as one component, but an aromatic polyamine included with TEP may also be used. TEP and the aromatic polyamine are not particularly limited as long as TEP and the aromatic polyamine interact with each other through a weak bond such as a hydrogen bond to form a crystal lattice, and the salts thereof are also included. As the adopted aromatic polyamine, preferably, 4,4'-diaminodiphenylmethane, 3,3'-diaminodiphenylmethane or the like may be exemplified.

The ratio between TEP and the aromatic polyamine forming the clathrate compound is not particularly limited as long as the ratio allows the clathrate compound to be formed; however, the ratio is preferably such that with respect to 1 mol of TEP, the amount of the aromatic polyamine is preferably 0.1 to 5.0 mol and more preferably 0.5 to 3.0 mol. The clathrate compound may further include a third component, and in such a case, the content of the third component is preferably 40 mol% or less and more preferably 10 mol% or less with respect to the total amount of the clathrate compound, and the clathrate compound most preferably includes no third component.

The clathrate compound used in the present invention may be obtained in a high yield by directly mixing or kneading TEP and the aromatic polyamine with each other, or by mixing TEP and the aromatic polyamine with each other in a solvent. When a solvent is used, TEP and the aromatic polyamine were added to the solvent, then subjected to a heat treatment or a heat reflux treatment, if necessary, while being stirred, and subsequently the clathrate compound may be obtained by precipitation. The solvent is not particularly limited, but methanol and ethyl acetate may be preferably used.

The formation of the clathrate compound may be verified by, for example, TG-DSC, infrared absorption spectra (IR), XRD, solid NMR spectra, and X-ray structure analysis. In addition, the composition and the proportions of the clathrate compound may be verified by, for example, thermal analysis, ¹H-NMR spectra, high performance liquid chromatography (HPLC) and elemental analysis.

### (Other Components)

Moreover, if necessary, various known additives may be mixed such as a curing agent, a curing accelerator, a plasticizer, an extender, a filler, a reinforcing agent, a pigment, a flame retardant, a thickening agent, a modifier and a release agent.

### (Mixing Proportions)

In the epoxy resin composition of the present invention, the proportion of the glycidylamine-type epoxy resin is preferably within a range from 10 to 100% by weight, more preferably 10 to 60% by weight and furthermore preferably 20 to 50% by weight with respect to the amount of the whole of the epoxy resins used. When the proportion of the glycidylamine-type epoxy resin is less than 10% by weight, sometimes the self-adhesiveness is insufficient or the heat resistance is poor.

The amount of the aromatic polyamine used in the present invention falls preferably within a range from 1.0 to 40.0 parts by weight and more preferably within a range from 4.0 to 28.0 parts by weight with respect to 100 parts by weight of the epoxy resin. In general, the amine-based curing agent is used in an amount to provide an active hydrogen equivalent (stoichiometric amount) equal to the epoxy equivalent of the epoxy resin, but an amount equal to or less than the stoichiometric amount is sufficient in the epoxy resin composition of the present invention.

The amount of TEP used in the present invention falls preferably within a range from 0.1 to 10 mol and more preferably within a range from 0.3 to 2.0 mol with respect to 1 mol of the aromatic polyamine included in the epoxy resin composition. In the case where the aromatic polyamine and TEP form a clathrate compound, the mixing ratio between the aromatic polyamine and TEP is the ratio corresponding to the clathrate ratio.

### (Composition of Present Invention and Production of Cured Product Thereof)

The epoxy resin composition of the present invention is obtained by mixing the glycidylamine-type epoxy resin, the aromatic polyamine, TEP and other components, if necessary, or the glycidylamine-type epoxy resin, the clathrate compound between the aromatic polyamine and TEP, and other components, if necessary, and by mixing or kneading the resulting mixture at room temperature so as for the respective components to be sufficiently dispersed. In the mixing or kneading, a stirrer such as a kneader is optionally used, or a spatula or the like is also optionally used, or alternatively the resulting mixture is also optionally heated to be melted at a temperature not causing thickening and gelation in such a way that a sufficiently mixed state is formed.

The cured product of the present invention is a product obtained by heat treating and curing the epoxy resin composition. The curing temperature depends on the components to be mixed and purposes for mixing, but is preferably within a range of 120 to 320°C. When the reaction temperature exceeds 320°C, a structure different from the target structure is obtained due to the self-ring-opening reaction of the epoxy resin itself, and sometimes the glass transition temperature or the mechanical property such as the modulus of elasticity is degraded. On the other hand, when the reaction temperature is lower than 120°C, sometimes the addition reaction does not sufficiently proceed. The reaction time is usually falls within a range from 2 to 12 hours. By using a plurality of epoxy resins and a plurality of curing agents, the curing temperature and the physical properties in accordance with the object may be obtained.

### (Mode of Use)

The epoxy resin composition of the present invention can be suitably used, in combination with reinforcing fiber, for the production of prepregs and fiber-reinforced composite materials. For example, a prepreg can be prepared by known methods such as a method (a wet method) in which the epoxy resin composition to be used as the matrix resin is dissolved in a solvent such as methyl ethyl ketone or methanol so as to have a low viscosity, and the resulting solution is impregnated into reinforcing fiber, and a hot melt method (a dry method) in which the matrix resin is heated so as to have a low viscosity, and the resulting melt is impregnated into reinforcing fiber.

The epoxy resin composition of the present invention can also be suitably used in the applications to adhesiveness, semiconductor-sealing materials, laminated plates for printed wiring boards, varnishes, powder coating materials, casting materials and inks, in addition to prepregs and fiber-reinforced composite materials.

Hereinafter, the present invention will be explained below referring to Examples, but the technical scope of the present invention is not intended to be limited to these Examples.

### Examples

### [Analysis Methods]

### <Thermogravimetric Measurement/Differential Scanning Calorimetry (TG-DSC) >

The measurement was performed by using a thermogravimetric measurement apparatus (trade name: TGA-DSC1, manufactured by Mettler-Toledo International Inc.), with approximately 3 mg of crystals placed in an aluminum vessel, under nitrogen purge (the flow rate of nitrogen: 50 mL/min), at a programmed temperature increase rate of 20°C/min, in a measurement temperature range from room temperature to 500°C.

### <Differential Scanning Calorimetry (DSC)>

The measurement was performed by using a differential scanning calorimeter (trade name: DSC1, manufactured by Mettler-Toledo International Inc.), with approximately 8 mg of crystals placed in an aluminum vessel, under nitrogen purge (the flow rate of nitrogen: 50 mL/min), at a programmed temperature increase rate 10°C/min, in a measurement temperature range from 30°C to 350°C.

### Example 1

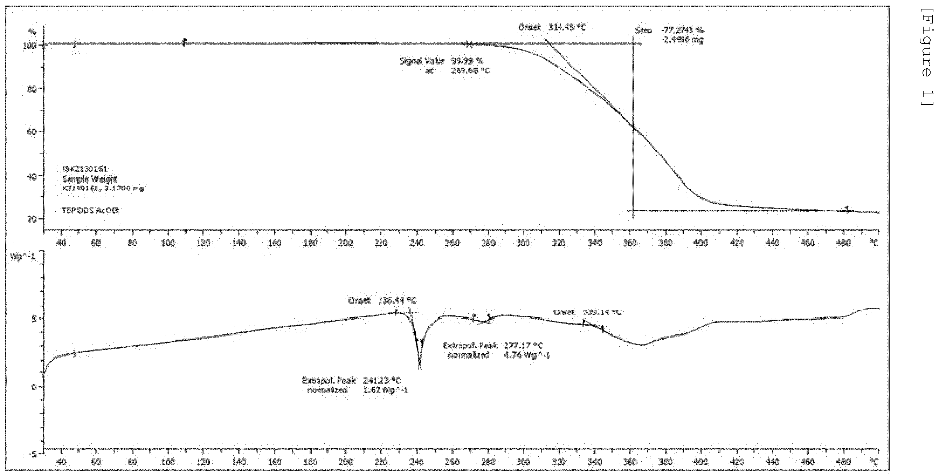

To 40.0 g of TEP (trade name: TEP-DF, manufactured by Asahi Yukizai Corporation), 200 ml of ethyl acetate was added, the resulting mixture was stirred, a solution composed of 49.9 g of 4,4'-DDS and 100 ml of ethyl acetate was added dropwise to the mixture at room temperature, and the resulting mixture was stirred after the addition for 3 hours under reflux. After cooling, filtration and vacuum drying were performed, and thus a clathrate compound (A-1) composed of TEP and 4,4'-DDS (molar ratio was 1:2) was obtained at a yield of 91.40. The obtained (A-1) was subjected to a TG-DSC measurement, and the results thus obtained are shown in Figure 1. From Figure 1, the release temperature of 4,4'-DDS was found to be 241.2°C.

### Example 2

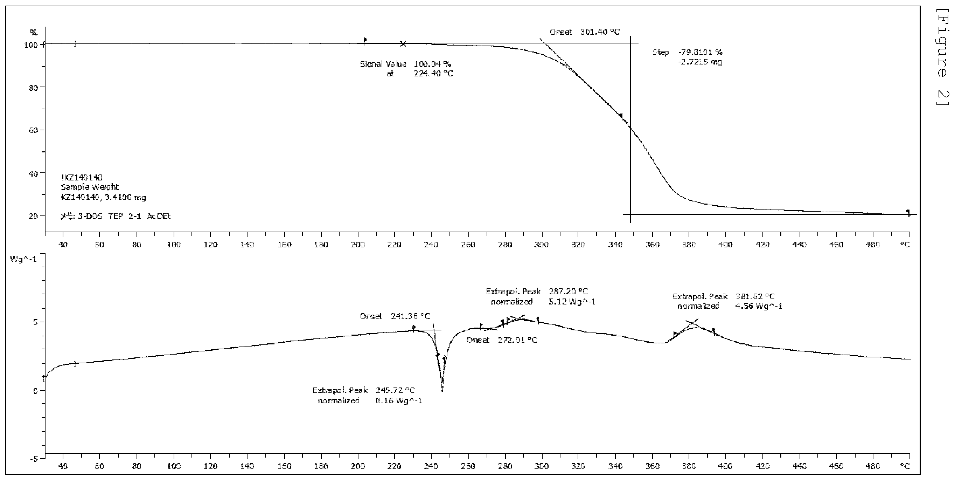

To 4.00 g of TEP, 20 ml of ethyl acetate was added, the resulting mixture was stirred, a solution composed of 4.99 g of 3,3'-DDS and 20 ml of ethyl acetate was added dropwise to the mixture at room temperature, and the resulting mixture was stirred after the addition for 3 hours under reflux. After cooling, filtration and vacuum drying were performed, and thus a clathrate compound (A-2) composed of TEP and 3,3'-DDS (molar ratio was 1:2) was obtained at a yield of 90.4%. The obtained (A-2) was subjected to a TG-DSC measurement, and the results thus obtained are shown in Figure 2. From Figure 2, the release temperature of 4,4'-DDS was found to be 245.7°C.

### Example 3 to Example 6

With the mixing proportions shown in Table 1, 2.5 g of TGDDM and the clathrate compound (A-1) or (A-2) were kneaded at room temperature for 10 minutes, and thus the epoxy resin compositions (B-1) to (B-4) were prepared.

**[Table 1]**

| | Epoxy resin composition | Clathrate compound (A-1)*¹ | Clathrate compound (A-2)*¹ |
|---|---|---|---|
| Example 3 | (B-1) | 0.180g (4) | - |
| Example 4 | (B-2) | 0.540g (12) | - |
| Example 5 | (B-3) | 1.26g (28) | - |
| Example 6 | (B-4) | - | 0.180g (4) |

| | | | |
|---|---|---|---|
| *1: The numerical values in the parentheses each represent the parts by weight of 4,4'-DDS or 3,3'-DDS contained in the clathrate compound with respect to the weight of the whole epoxy resin taken to be 100 in the resin composition (unit: phr). | | | |

### Example 7 to Example 12

With 2.5 g of TGDDM, 4,4'-DDS or 3,3'-DDS and TEP were kneaded in the mixing proportions shown in Table 2, at room temperature for 10 minutes, and thus the epoxy resin compositions (B-5) to (B-10) were prepared.

**[Table 2]**

| | Epoxy resin composition | 4, 4'-DDS*¹ | 3, 3'-DDS*¹ | TEP*² |
|---|---|---|---|---|
| Example 7 | (B-5) | 0.10g (4) | - | 0.08g (0.5) |
| Example 8 | (B-6) | 0.10g (4) | - | 0.16g (1) |
| Example 9 | (B-7) | 0.10g (4) | - | 0.24g (1.5) |
| Example 10 | (B-8) | 0.30g (12) | - | 0.24g (0.5) |
| Example 11 | (B-9) | 0.70g (28) | - | 0.56g (0.5) |
| Example 12 | (B-10) | - | 0.10g (4) | 0.08g (0.5) |

| | | | | |
|---|---|---|---|---|
| *1: The numerical values in the parentheses each represent the parts by weight of 4,4'-DDS or 3,3'-DDS with respect to the weight of the whole epoxy resin taken to be 100 in the resin composition (unit: phr). *2 The numerical values in the parentheses each represent the number of moles of TEP used with respect to 1 mol of DDS used. | | | | |

### Comparative Example 1 to Comparative Example 4

The epoxy resins (CB-1) to (CB-4) were prepared in the same manner as in Example 7, Example 10, Example 11 and Example 12, respectively, except that TEP was not used.

A fraction of each of the obtained epoxy resin compositions (B-1) to (B-10) and (CB-1) to (CB-4) was sampled, and the exothermic heats of the epoxy resin compositions based on the curing reaction were measured by using the differential scanning calorimeter. As the results thus obtained, the respective curing onset temperatures, peak temperatures of the reaction heats and heat quantities (the integrated heats per 1 part by weight in terms of the weight exclusive of the weight of the added TEP) in the range of 100°C or higher and 350°C or lower are shown in Table 3.

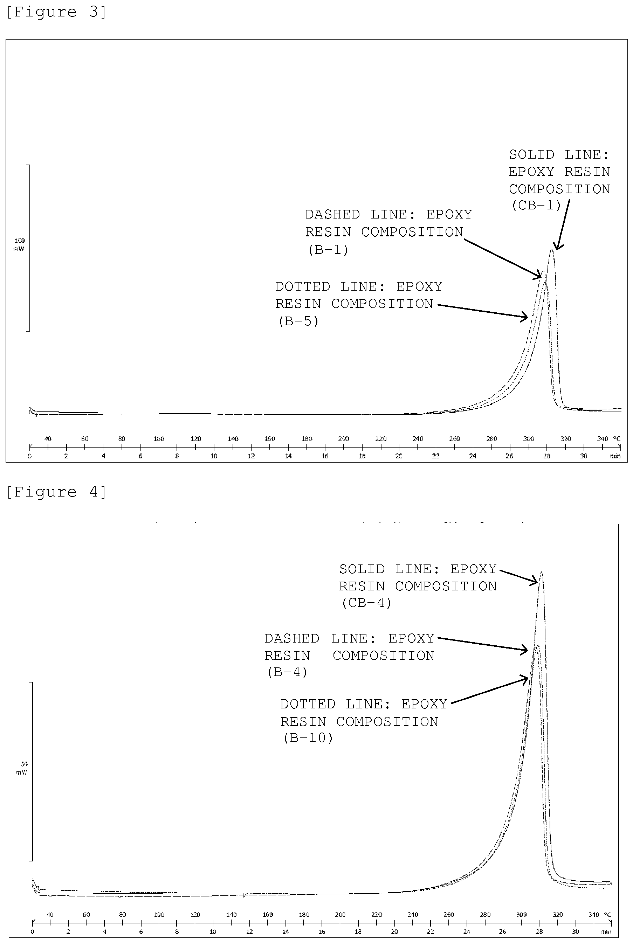

The DSC charts of the epoxy resin compositions (B-1), (B-5) and (CB-1) are shown in Figure 3, and the DSC charts of the epoxy resin compositions (B-4), (B-10) and (CB-4) are shown in Figure 4. In each of Figures 3 and 4, the abscissa represents the measurement temperature (°C), and the ordinate represents the exothermic heat quantity (heat flow/mW).

**[Table 3]**

| Epoxy resin composition | Curing onset temperature (°C) | Peak temperature of reaction heat (°C) | Heat quantity (J/g) | Peak temperature difference of reaction heat from peak temperature in non-addition of TEP (°C) | Heat quantity difference from heat quantity in non-addition of TEP (J/g) |
|---|---|---|---|---|---|
| (B-1) | 294.0 | 313.2 | 957.3 | -5.4 | - |
| (B-2) | - | 291.1 | - | -8.1 | - |
| (B-3) | - | 255.8 | - | -15.6 | - |
| (B-4) | 292.5 | 312.2 | 888.9 | - | - |
| (B-5) | 294.7 | 313.8 | 979.0 | -4.8 | -26.2 |
| (B-6) | 286.7 | 308.5 | 951.8 | - | -53.4 |
| (B-7) | 281.3 | 305.1 | 876.8 | - | -128.4 |
| (B-8) | - | 290.8 | - | -8.4 | - |
| (B-9) | - | 255.2 | - | -16.2 | - |
| (B-10) | 293.6 | 313.4 | 889.0 | - | - |
| (CB-1) | 302.0 | 318.6 | 1005.2 | - | - |
| (CB-2) | - | 299.2 | - | - | - |
| (CB-3) | - | 271.4 | - | - | - |
| (CB-4) | 298.7 | 316.6 | 1001.1 | - | - |

From the results of Table 3, it has been found that as compared with the epoxy resin compositions not including anything added thereto, the epoxy resin compositions including TEP added thereto undergo the decrease of the curing onset temperature, the decrease of the peak temperature of the reaction heat and the decrease of the heat quantity, and with the increase of the added amount of TEP, in particular the extent of decrease of the heat quantity is increased. In other words, the epoxy resin composition of the present invention can be cured at a lower temperature and with a smaller energy quantity. Moreover, it has also been revealed that even an addition of a curing agent in an amount equal to or smaller than the stoichiometric amount (4 to 28 phr) allows the curing reaction to proceed.

The evaluation of the storage stability was performed by storing the obtained epoxy resin compositions (B-3), (B-9) and (CB-3) at 40°C, and by measuring the number of days until the solidification was visually verified. The results thus obtained are shown in Table 4.

**[Table 4]**

| Epoxy resin composition | Number of days until solidification |
|---|---|
| (B-3) | Not solidified in 94 days |
| (B-9) | Solidified in 6 to 13 days |
| (CB-3) | Solidified in 48 days |

From the results shown in Table 4, it has been found that the epoxy resin composition (B-3) including the clathrate compound between TEP and 4,4'-DDS added thereto is longer in the number of days until the solidification than the epoxy resin composition (CB-3) not using TEP, and is improved in the one-pack stability. On the other hand, it has been found that the epoxy resin composition (B-9) including TEP and 4,4'-DDS added without forming a clathrate compound is solidified faster than the epoxy resin composition (CB-3) not using TEP, and accordingly TEP has a curing acceleration effect.

## Claims

1. An epoxy resin composition comprising a glycidylamine-type epoxy resin, an aromatic polyamine and 1,1,2,2-tetrakis(4-hydroxyphenyl)ethane.

2. The epoxy resin composition according to claim 1, wherein the aromatic polyamine and 1,1,2,2-tetrakis(4-hydroxyphenyl)ethane form a clathrate compound.

3. The epoxy resin composition according to claim 1 or 2, wherein the aromatic polyamine is 4,4'-diaminodiphenyl sulfone and/or 3,3'-diaminodiphenyl sulfone.

4. The epoxy resin composition according to claim 1 or 2, wherein the glycidylamine-type epoxy resin is a tri- or higher functional glycidylamine-type epoxy resin.

5. The epoxy resin composition according to claim 4, wherein the tri- or higher functional glycidylamine-type epoxy resin is N,N,N',N'-tetraglycidyl-4,4'-diaminodiphenylmethane.

6. A cured product obtained by curing the epoxy resin composition according to any one of claims 1 to 5.

7. A clathrate compound comprising (A) 4,4'-diaminodiphenyl sulfone and/or 3,3'-diaminodiphenyl sulfone and (B) 1,1,2,2-tetrakis(4-hydroxyphenyl)ethane.
